# EUROPÄISCHE PATENTANMELDUNG

(11) **EP 3 058 984 A1**
(43) Veröffentlichungstag der Anmeldung: **24.08.2016**
(21) Anmeldenummer: 15155989.5
(22) Anmeldetag: 20.02.2015
(51) Int. Cl.: A61N 1/375, C04B 35/622

(54) **Bauteil mit einem keramischen Grundkörper, mit einem Leitungselement und mit einem Befestigungselement und ein Verfahren zu dessen Herstellung**

(71) Anmelder: Heraeus Deutschland GmbH & Co. KG, 63450 Hanau (DE)
(72) Erfinder: Trötzschel, Jens, 63549 Ronneburg (DE); Hausch, Ulrich, 60318 Frankfurt (DE)
(74) Vertreter: Herzog, Fiesser & Partner Patentanwälte PartG mbB

(57) **Zusammenfassung**

Die Anmeldung betrifft ein Bauteil beinhaltend
i. einen Grundkörper (10) mit einer ersten Bauteiloberfläche (40) und einer weiteren Bauteiloberfläche (50), wobei der Grundkörper mindestens zu 50 Gew.-%, bezogen auf das Gesamtgewicht des Grundkörpers, eine Keramik beinhaltet,
ii. mindestens ein elektrisches Leitungselement (30), wobei das mindestens eine elektrische Leitungselement mindestens zu 51 Gew.-%, bezogen auf das Gesamtgewicht des elektrischen Leitungselements, ein Metall beinhaltet und wobei das mindestens eine elektrische Leitungselement durch den gesamten Grundkörper hindurch von der ersten Bauteiloberfläche zu der weiteren Bauteiloberfläche führt und
iii. mindestens ein Befestigungselement (20) mit einer Kontaktfläche (60), wobei das mindestens eine Befestigungselement mindestens zu 51 Gew.-%, bezogen auf das Gesamtgewicht des Befestigungselements, ein Metall beinhaltet und wobei das Befestigungselement mindestens zu einem Teil von dem Grundkörper umgeben ist.

Weiterhin betrifft die Anmeldung eine Vorrichtung beinhaltend ein Gehäuse und das Bauteil. Die Anmeldung betrifft darüber hinaus auch ein Verfahren zur Herstellung des Bauteils.

## Beschreibung

Die Erfindung betrifft ein Bauteil beinhaltend i. einen Grundkörper mit einer ersten Bauteiloberfläche, und einer weiteren Bauteiloberfläche, wobei der Grundkörper mindestens zu 50 Gew.-%, bezogen auf das Gesamtgewicht des Grundkörpers, eine Keramik beinhaltet; ii. mindestens ein elektrisches Leitungselement, wobei das mindestens eine elektrische Leitungselement mindestens zu 51 Gew.-%, bezogen auf das elektrische Leitungselement, ein Metall beinhaltet, wobei das mindestens eine elektrische Leitungselement durch den gesamten Grundkörper hindurch von der ersten Bauteiloberfläche zu der weiteren Bauteiloberfläche führt; iii. mindestens ein Befestigungselement mit einer Kontaktfläche, wobei das mindestens eine Befestigungselement mindestens zu 51 Gew.-%, bezogen auf das Befestigungselement, ein Metall beinhaltet, wobei das Befestigungselement mindestens zu einem Teil von dem Grundkörper umgeben ist.

Weiterhin betrifft die Erfindung eine Vorrichtung beinhaltend a. ein Gehäuse, das einen Innenbereich von einem Außenbereich mindestens zu einem Teil trennt, beinhaltend: i). eine Gehäusewand mit einer ersten Gehäuseoberfläche, die dem Innenbereich zugewandt ist und einer weiteren Gehäuseoberfläche, die dem Außenbereich des Gehäuses zugewandt ist; ii). mindestens eine Aussparung in der Gehäusewand; b. ein erfindungsgemäßes Bauteil. Die Erfindung betrifft auch ein Verfahren zur Herstellung eines Bauteils mit den Schritten: I. Bereitstellen einer ersten Masse zur Formung eines ersten Teilbereiches; II. Bereitstellen einer weiteren Masse zur Formung mindestens eines weiteren Teilbereiches; III. Bereitstellen einer dritten Masse zur Formung eines dritten Teilbereiches; IV. Bilden eines Bauteilvorläufers, wobei ein erster Vorläufer für einen Grundkörper aus dem ersten Teilbereich gebildet wird, wobei ein weiterer Vorläufer für ein elektrisches Leitungselement aus dem mindestens einen weiteren Teilbereich gebildet wird und wobei ein dritter Vorläufer für mindestens ein Befestigungselement aus dem dritten Teilbereich gebildet wird; V. Behandeln des Bauteilvorläufers bei einer Temperatur in einem Bereich von 500 bis 2500 °C.

Bauteile mit keramischem Grundkörper und integriertem Leitungselementen werden oft bei medizinischen Geräten eingesetzt, wenn eine elektrische Verbindung durch ein biokompatibles Material hindurch erzeugt werden soll. Vor allem bei der Therapie von Herzfehlern, wie beispielsweise bei der Verwendung von Herzschrittmachern werden solche Bauteile verwendet.

Beim Einsatz solcher Bauteile in beispielsweise ein Gehäuse eines Herzschrittmachers können unterschiedliche Anforderungen an das Material und die Verbindung zwischen Material und Gehäuse gestellt sein. Ein Beispiel für eine solche Verbindung ist in der DE 10 2009 035 971 A1 gezeigt, bei der ein Bauteil in Form einer elektrischen Durchführung mit einem ringförmigen Haltelement versehen ist, das eine Durchgangsöffnung aufweist, in die ein Isolationselement und ein Leitungsdraht eingebracht sind.

Auf das ringförmige Haltelement der DE 10 2009 035 971 A1 wird an der Verbindung der elektrischen Durchführung mit einem Gehäuse ein großer Zug in alle drei Raumrichtungen ausgeübt. Das führt zu sehr hohen Spannungen zwischen der Durchführung und dem diese umgebenden ringförmigen Haltelement. Es können Risse in der Durchführung ausgebildet werden. Auch der Ring selbst kann zum Beispiel durch Risse oder Verformung undicht werden, oder gar brechen.

Allgemein liegt eine Aufgabe der vorliegenden Erfindung darin, die sich aus dem Stand der Technik ergebenden Nachteile zumindest teilweise zu überwinden.

Eine weitere Aufgabe besteht darin, ein Bauteil bereitzustellen, das eine einfache Anbindung an weitere Vorrichtungen ermöglicht, ohne dabei zu viel Spannung auf das Bauteil oder die Verbindung auszuüben.

Weiterhin besteht eine Aufgabe darin, ein Bauteil bereitzustellen, das so ausgestaltet ist, dass Kräfte, die bei der Verbindung des Bauteils mit einem weiteren Element, wie einem Gehäuse, auftreten, möglichst effektiv abgeleitet werden können.

Es ist eine weitere Aufgabe, ein Bauteil bereitzustellen, das eine möglichst lange Lebensdauer aufweist, insbesondere in sauren Lösungen oder unter Einwirkung von einem hohem Druck, oder beidem.

Darüber hinaus ist es eine Aufgabe, ein Bauteil bereitzustellen, das kostengünstig und in möglichst wenigen Schritten herstellbar ist.

Eine weitere Aufgabe besteht darin, eine Vorrichtung mit einem Bauteil bereitzustellen, das eine möglichst lange Lebensdauer aufweist, insbesondere in sauren Lösungen oder unter Einwirkung von hohem Druck, oder beidem.

Weiterhin ist es eine Aufgabe, ein Verfahren zur Herstellung des Bauteils bereitzustellen, das kostengünstig und effizient ist.

Des Weiteren ist es eine Aufgabe, ein Verfahren zur Herstellung eines Bauteils bereitzustellen, das zu einem Bauteil führt, das eine möglichst lange Lebensdauer insbesondere in sauren Lösungen oder unter hohem Druck aufweist.

Eine weitere Aufgabe besteht darin, eine Vorrichtung bereitzustellen, die eine möglichst spannungsfreie Verbindung zwischen dem Bauteil und einem Gehäuse aufweist.

Einen Beitrag zur Lösung mindestens einer der eingangs genannten Aufgaben leisten die Gegenstände der kategoriebildenden Ansprüche. Die Gegenstände der von den kategoriebildenden Ansprüchen abhängigen Unteransprüche stellen bevorzugte Ausgestaltungen dar.

Ein erster Gegenstand der vorliegenden Erfindung ist ein Bauteil, beinhaltend:
i. einen Grundkörper mit einer ersten Bauteiloberfläche, und einer weiteren Bauteiloberfläche, wobei der Grundkörper mindestens zu 50 Gew.-%, bezogen auf das Gesamtgewicht des Grundkörpers, eine Keramik beinhaltet;
ii. mindestens ein elektrisches Leitungselement, wobei das mindestens eine elektrische Leitungselement mindestens zu 51 Gew.-%, bezogen auf das elektrische Leitungselement, ein Metall beinhaltet,
   wobei das mindestens eine elektrische Leitungselement durch den gesamten Grundkörper hindurch von der ersten Bauteiloberfläche zu der weiteren Bauteiloberfläche führt;
iii. mindestens ein Befestigungselement mit einer Kontaktfläche, wobei das mindestens eine Befestigungselement mindestens zu 51 Gew.-%, bezogen auf das Gesamtgewicht des Befestigungselements, ein Metall beinhaltet;
   wobei das Befestigungselement mindestens zu einem Teil von dem Grundkörper umgeben ist.

Der Grundkörper kann jede Komponente enthalten, die der Fachmann dazu geeignet findet in einem erfindungsgemäßen Bauteil verwendet zu werden. Insbesondere, wenn das Bauteil in eine Vorrichtung zum Implantieren in einen Körper eingesetzt werden soll, sollte das Bauteil Komponenten beinhalten, die sich beim Kontakt mit Körperflüssigkeit nicht in ihrer Form und Zusammensetzung verändern oder auflösen. Weiterhin sollte der Grundkörper aus einem Material bestehen, das elektrischen Strom wenig bis gar nicht leitet. Bevorzugt weist der Grundkörper isolierende Eigenschaften gegenüber dem durchgeführten elektrischen Leitungselement auf. Bevorzugt beinhaltet der Grundkörper eine erste Komponente ausgewählt aus der Gruppe bestehend aus einem Polymer, ein Glas, einer Keramik, einem Cermet oder einer Mischung aus mindestens zwei hieraus.

Das Polymer kann ausgewählt sein aus der Gruppe bestehend aus einem Polyacrylat, wie Polymethylmethycrylat (PMMA), einem Polyurethan, einem Polyethylen, wie Polytetrafluorethylen (PTFE) einem Polycarbonat oder einer Mischung aus mindestens zwei hiervon.

Die Keramik kann ausgewählt sein aus der Gruppe bestehend aus einer Oxidkeramik, einer Silikatkeramik, einer Nichtoxid-Keramik oder einer Mischung aus mindestens zwei davon. Die Oxidkeramik ist bevorzugt ausgewählt aus der Gruppe bestehend aus einem Metalloxid, einem Halbmetalloxid oder einer Mischung davon. Das Metall des Metalloxids kann ausgewählt sein aus der Gruppe bestehend aus Aluminium, Barium, Calcium, Magnesium, Natrium, Kalium, Eisen, Zirkonium, Titan oder einer Mischung von mindestens zwei davon. Das Metalloxid ist bevorzugt ausgewählt aus der Gruppe bestehend aus Aluminiumoxid (Al₂O₃), Magnesiumoxid (MgO), Zirkoniumoxid (ZrO₂) Yttriumoxid (Y₂O₃), Aluminiumtitanat (Al₂TiO₅) oder einer Mischung von mindestens zwei hiervon. Das Halbmetall des Halbmetalloxids ist bevorzugt ausgewählt aus der Gruppe bestehend aus Bor, Silicium, Tellur oder einer Mischung von mindestens zwei davon.

Die Silikatkeramik ist bevorzugt ausgewählt aus der Gruppe bestehend aus einem Steatit (Mg₃[Si₄O₁₀(OH)₂]), Cordierit (Mg,Fe²⁺)₂(Al₂Si)[Al₂Si₄O₁₈]), Mullit (Al₂Al₂₊₂ₓSi₂₋₂ₓO₁₀₋ₓ mit x = Sauerstoffleerstellen pro Elementarzelle), Feldspat (Ba,Ca,Na,K,NH₄)(Al,B,Si)₄O₈) oder einer Mischung aus mindestens zwei davon.

Die Nichtoxid-Keramik kann ausgewählt sein aus der Gruppe bestehend aus einem Carbid, einem Nitrid oder einer Mischung daraus. Das Carbid kann ausgewählt sein aus der Gruppe bestehend aus Siliciumcarbid (SiC), Borcarbid (B₄C), Titancarbid (TiC), Wolframcarbid, Zementit (Fe3C). Das Nitrid kann ausgewählt sein aus der Gruppe bestehend aus Siliciumnitrid (Si₃N₄), Aluminiumnitrid (AlN), Siliciumaluminiumoxinitrid (SIALON) oder einer Mischung aus mindestens zwei hiervon.

Der Grundkörper beinhaltet bevorzugt eine Oxidkeramik, bevorzugt Aluminiumoxid (Al₂O₃). Weiterhin bevorzugt beinhaltet der Grundkörper Aluminiumoxid in einer Menge in einem Bereich von 50 bis 100 Gew.-%, oder bevorzugt in einem Bereich von 70 bis 100 Gew.-%, oder bevorzugt in einem Bereich von 90 bis 100 Gew.-%, jeweils bezogen auf das Gesamtgewicht des Grundkörpers. Die Partikelgrößenverteilung des Grundkörpers, wie beispielsweise Aluminiumoxids, beträgt bevorzugt d₂₀ = 0,2 µm, d₅₀ = 0,4 µm, oder d₉₀ = 1 µm, oder eine Kombination von zwei oder mehr der vorgenannten. Das bedeutet, dass mindestens 20 % der gesamten Zahl der Partikel des Grundkörpers einen Durchmesser von 0,2 oder kleiner aufweisen, oder mindestens 50 % der Partikel einen Durchmesser von 0,4 oder kleiner aufweisen, oder mindestens 90 % der Partikel einen Durchmesser von 0,2 oder kleiner aufweisen. Weiter bevorzugt ist die Kombination von zwei oder mehr der vorgenannten Partikelgrößen. Die Partikelgrößen können auf verschiedene Arten bestimmt werden. Bevorzugt werden hierzu optische Methoden verwendet, wie elektrische Lichtstreuung, Kondensationskernzählung oder Laserbeugung. Bevorzugt wird die Partikelgröße mittels Laserbeugung, gemäß der erläuterten Methode in den Testmethoden, bestimmt. Die Summe aller Bestandteile des Grundkörpers ergibt stets 100 Gew.-%.

Im Rahmen der Erfindung wird als "Cermet" ein Verbundwerkstoff aus einem oder mehreren keramischen Werkstoffen in mindestens einer metallischen Matrix oder ein Verbundwerkstoff aus einem oder mehreren metallischen Werkstoffen in mindestens einer keramischen Matrix verstanden. Zur Herstellung eines Cermets kann beispielsweise ein Gemisch aus mindestens einem keramischen Pulver und mindestens einem metallischen Pulver verwendet werden, welches beispielsweise mit mindestens mit einem Bindemittel und gegebenenfalls mindestens einem Lösungsmittel versetzt werden kann. Eine Auswahl für die keramischen Bestandteile und die metallischen Bestandteile des Cermets können sich aus denen zusammensetzen, die für den Grundkörper, das elektrische Leitungselement oder das Befestigungselement angegeben wurden.

Der Grundkörper des erfindungsgemäßen Bauteils kann jede Form aufweisen, die der Fachmann für den Einsatz in einem Bauteil auswählen würde. Der Grundkörper kann beispielsweise eine Form aufweisen ausgewählt aus der Gruppe bestehend aus kugelförmig, zylinderförmig, quaderförmig, kegelförmig, pyramidenförmig, eiförmig, scheibenförmig, oder einer Kombination aus mindestens zwei hieraus. Bevorzugt ist der Grundkörper als Zylinder ausgestaltet.

Durch mindestens einen Teil des Grundkörpers erstreckt sich das mindestens eine elektrische Leitungselement. Das mindestens eine elektrische Leitungselement kann jede Form aufweisen, die der Fachmann zur Durchleitung von elektrischem Strom durch den Grundkörper verwenden würde. Das mindestens eine elektrische Leitungselement weist bevorzugt eine Form ausgewählt aus der Gruppe bestehend aus drahtförmig, quaderförmig, kegelförmig oder einer Kombination aus mindestens zwei davon. Bevorzugt weist das mindestens eine elektrische Leitungselement eine längliche Form auf. Weiter bevorzugt variieren die Querschnittsflächen des länglichen Leitungselements senkrecht zur Längsausdehnung entlang des Leitungselements. Bevorzugt liegt dabei ein wiederkehrendes Muster an größeren und kleineren Querschnittsflächen vor. Ein längliches Leitungselement mit einem solchen wiederkehrenden Muster wird auch als "bambusförmig" beschrieben.

Das mindestens eine elektrische Leitungselement weist ein erstes und ein weiteres Ende auf. Als Enden des elektrischen Leitungselements werden bevorzugt Bereiche bezeichnet, die aus dem Grundkörper herausragen oder mit einer Ebene oder Oberfläche des Grundkörpers abschließen. Die Enden sind nicht vom Grundkörper vollständig umgeben. Das erste Ende des elektrischen Leitungselementes ist von der ersten Bauteiloberfläche des Grundkörpers mindestens zum Teil umgeben, während das weitere Ende des elektrischen Leitungselementes mindestens zum Teil von der weiteren Bauteiloberfläche des Grundkörpers umgeben ist. Die Bauteiloberflächen können unterschiedlich große Flächen des Grundkörpers bedecken oder auch gleich große Flächen. Die erste und die weitere Bauteiloberfläche weisen bevorzugt in unterschiedliche Richtungen vom Bauteil weg. Weiterhin bevorzugt weisen die erste und die weitere Bauteiloberfläche in entgegengesetzte Richtungen vom Bauteil weg. Die erste und die weitere Bauteiloberfläche können durch mindestens eine dritte Bauteiloberfläche mindestens zum Teil von einander getrennt sein.

In einer erfindungsgemäßen Ausgestaltung ragen die beiden Enden des mindestens einen elektrischen Leitungselementes in entgegengesetzte Richtungen aus dem Grundkörper heraus. Oftmals ragen die Enden auch in nicht entgegengesetzten Richtungen aus dem Grundkörper heraus. In diesen Fällen kann ein Winkel gelegt werden zwischen einer Längsachse, die durch ein erstes Ende eines elektrischen Leitungselementes geht und einer zweiten Längsachse, die durch ein weiteres Ende des elektrischen Leitungselementes geht. Der Winkel zwischen der Längsachse des ersten Endes des mindestens einen elektrischen Leitungselements und der Längsachse des weiteren Endes des elektrischen Leitungselements liegt dann bevorzugt in einem Bereich von 90 bis 180 °, oder bevorzugt in einem Bereich von 100 bis 180 °, oder bevorzugt in einem Bereich von 110 bis 180 °.

In einer weiteren erfindungsgemäßen Ausgestaltung schließen die beiden Enden des mindestens einen elektrischen Leitungselementes mit einer der Außenflächen, vorzugsweise beiden Flächen, des Grundkörpers ab. In einer weiteren bevorzugten Ausführungsform verlaufen mehrere Leitungselemente nicht parallel durch den Grundkörper. Bevorzugt kann ein Winkel gelegt werden zwischen einer ersten Längsachse, die durch ein erstes Ende eines elektrischen Leitungselementes geht und einer zweiten Längsachse, die durch ein weiteres Ende des elektrischen Leitungselementes geht. Der Winkel zwischen der Längsachse des ersten Endes des mindestens einen elektrischen Leitungselements und der Längsachse des weiteren Endes des elektrischen Leitungselements liegt dann bevorzugt in einem Bereich von 90 bis 180 °, oder bevorzugt in einem Bereich von 100 bis 180 °, oder bevorzugt in einem Bereich von 110 bis 180 °.

Das mindestens eine elektrische Leitungselement beinhaltet mindestens ein Metall bevorzugt in einem Bereich von 51 bis 100 Gew.-%, oder bevorzugt in einem Bereich von 60 bis 100 Gew.-%, oder bevorzugt in einem Bereich von 70 bis 100 Gew.-%, oder 75 bis 95 Gew.-%, oder 80 bis 95 Gew.-%, bezogen auf das Gesamtgewicht des elektrischen Leitungselements.

Das elektrische Leitungselement beinhaltet bevorzugt mindestens eine weitere Komponente. Die mindestens eine weitere Komponente kann ausgewählt sein aus der Gruppe bestehend aus einer Keramik, einem Glas, einem Cermet und einem Polymer oder einer Mischung aus mindestens zwei hieraus. Die Keramik oder das Polymer sind bevorzugt aus der gleichen Gruppe ausgewählt wie die des Grundkörpers. Das elektrische Leitungselement beinhaltet dann bevorzugt die weitere Komponente in einem Bereich von 1 bis 49 Gew.-%, oder bevorzugt in einem Bereich von 3 bis 40 Gew.-%, oder bevorzugt in einem Bereich von 5 bis 30 Gew.-%, und das mindestens eine Metall in einem Bereich von 51 bis 99 Gew.-%, oder bevorzugt in einem Bereich von 60 bis 97 Gew.-%, oder bevorzugt in einem Bereich von 70 bis 95 Gew.-%, jeweils bezogen auf das Gesamtgewicht des mindestens einen elektrischen Leitungselements. Die Summe aller Bestandteile des elektrischen Leitungselements ergibt stets 100 Gew.-%.

Das mindestens eine elektrische Leitungselement kann jede Form aufweisen, die der Fachmann für den Einsatz in einem Bauteil auswählen würde. Das mindestens eine elektrische Leitungselement kann rund, eckig oder oval in seiner Querschnittsfläche sein. Das elektrische Leitungselement weist bevorzugt eine längliche, runde Form auf. Bevorzugt erstreckt sich das elektrische Leitungselement mit seiner länglichen Ausdehnung von der ersten Bauteiloberfläche zu der weiteren Bauteiloberfläche. Insbesondere, wenn das Bauteil über das Befestigungselement mit einer weiteren Vorrichtung, wie beispielsweise einem Gehäuse eines medizinischen Gerätes, verbunden ist, bildet das elektrische Leitungselement eine elektrische Leitung vom Außenbereich des Gehäuses zum Innenbereich des Gehäuses aus.

Das mindestens eine Befestigungselement beinhaltet zu mindestens 51 Gew.-%, bevorzugt in einem Bereich von 51 bis 100 Gew.-%, oder bevorzugt in einem Bereich von 60 bis 98 Gew.-%, oder bevorzugt in einem Bereich von 80 bis 95 Gew.-%, jeweils bezogen auf das Befestigungselement, ein Metall. Weiterhin bevorzugt beinhaltet das Befestigungselement in einem Bereich von 1 bis 49 Gew.-%, oder bevorzugt in einem Bereich von 3 bis 45 Gew.-%, oder bevorzugt in einem Bereich von 5 bis 20 Gew.-%, jeweils bezogen auf das Gesamtgewicht des Befestigungselements eine weitere Komponente ausgewählt aus der Gruppe bestehend aus einem Polymer, eine Keramik und einem Cermet oder einer Mischung aus mindestens zwei hiervon. Das Polymer, die Keramik oder das Cermet können identisch zu denen sein, wie für den Grundkörper beschrieben. Die Summe aller Bestandteile des Befestigungselements ergibt stets 100 Gew.-%.

Das Befestigungselement ist erfindungsgemäß mindestens zu einem Teil von dem Grundkörper umgeben. Dabei steht mindestens ein Teil der Oberfläche des Grundkörpers mit einem Teil der Oberfläche des Befestigungselements in Kontakt und zumindest ein Teil des Befestigungselements ist in einer Aussparung des Grundkörpers angeordnet. Die Fläche, die durch den Kontakt zwischen Grundkörper und Befestigungskörper gebildet wird, wird als Verbindungsfläche bezeichnet. Die Fläche, die nicht mit dem Grundkörper in Kontakt steht oder von ihm umgeben ist, wird als Kontaktfläche bezeichnet. Die Kontaktfläche ist frei zugänglich, beispielsweise für einen Kontakt mit anderen Elementen, wie einem Gehäuse einer Vorrichtung.

In einer bevorzugten Ausgestaltung des Bauteils ist mindestens 50 %, bevorzugt mindestens 60 %, oder bevorzugt mindestens 70 % der Oberfläche des mindestens einen Befestigungselementes von dem Grundkörper umgeben. Bevorzugt ist die Oberfläche des mindestens einen Befestigungselements von dem Grundkörper in einem Bereich von 10 bis 90 %, oder bevorzugt in einem Bereich von 20 bis 85 %, oder bevorzugt in einem Bereich von 30 bis 80 % von dem Grundkörper umgeben.

Das mindestens eine Befestigungselement kann jede Form aufweisen, die der Fachmann für ein Befestigungselement in einem erfindungsgemäßen Bauteil verwenden würde. Die Form des mindestens einen Befestigungselements ist bevorzugt ausgewählt aus der Gruppe bestehend aus einer Kugel, einem Quader, einem Zylinder, einer Pyramide, einem runden Ring, einem eckigen Ring, einem offenen Ring, einem geschlossenen Ring oder einer Kombination aus mindestens zwei hieraus. Weiterhin bevorzugt ist das mindestens eine, sind bevorzugt alle Befestigungselemente als Quader ausgestaltet. Weiterhin bevorzugt erstreckt sich das mindestens eine Befestigungselement entlang mindestens eines Teils der Außenoberfläche des Grundkörpers. Das mindestens eine Befestigungselement weist mindestens eine Teilfläche der Oberfläche auf, die von der Außenseite des Bauteils zugänglich ist. Bevorzugt weist jedes Bauteil mindestens ein, insbesondere zwei, drei, vier oder fünf Befestigungselemente auf. Das Volumen aller an einem Bauteil vorgesehenen Befestigungselemente beträgt bevorzugt in einem Bereich von 0,001 mm³ bis 5 cm³, oder bevorzugt in einem Bereich von 1 mm³ bis 3 cm³, oder bevorzugt in einem Bereich von 5 mm³ bis 1 cm³, oder besonders bevorzugt in einem Bereich von 0,001 bis 400 mm³.

In einer bevorzugten Ausgestaltung des Bauteils ist das mindestens eine Befestigungselement ringförmig ausgestaltet. Bevorzugt erstreckt sich das mindestens eine ringförmige Befestigungselement entlang der Oberfläche des Bauteils. Weiterhin bevorzugt ist das mindestens eine Befestigungselement so ringförmig um den Grundkörper angeordnet, dass es die Oberfläche des Bauteils in eine erste Bauteiloberfläche und eine weitere Bauteiloberfläche unterteilt. Bevorzugt steht das mindestens eine Befestigungselement mit dem mindestens einen elektrischen Leitungselement nicht in direktem Kontakt. Bevorzugt ist das mindestens eine Befestigungselement durch einen Teil des Grundkörpers von dem mindestens einen elektrischen Leitungselement getrennt. Gemäß einer alternativen Ausgestaltung ist mindestens eines der Befestigungselemente mit mindestens einem elektrischen Leitungselement verbunden. Weiter bevorzugt befindet sich diese Verbindung im Innern des Bauteils.

In einer weiteren bevorzugten Ausgestaltung des Bauteils weist das Bauteil mehr als ein, oder bevorzugt mehr als zwei, oder bevorzugt mehr als drei Befestigungselemente auf. Die mehreren Befestigungselemente haben bevorzugt zu mindestens einer gleichen Bauteiloberfläche des Bauteils Kontakt. Weiterhin bevorzugt ist das mindestens eine Befestigungselement von der ersten Bauteiloberfläche umgeben.

In einer bevorzugten Ausgestaltung des Bauteils bildet das Befestigungselement keine Verbindung durch den Grundkörper hindurch von der ersten Bauteiloberfläche zu der weiteren Bauteiloberfläche. Das mindestens eine Befestigungselement erstreckt sich bevorzugt an keiner Stelle durch den Grundkörper des Bauteils hindurch. Das mindestens eine Befestigungselement steht bevorzugt mit mindestens der ersten oder der weiteren Bauteiloberflächen in Kontakt.

In einer bevorzugten Ausgestaltung des Bauteils ist der Grundkörper mit dem mindestens einen elektrischen Leitungselement und dem mindestens einen Befestigungselement stoffschlüssig verbunden. Eine stoffschlüssige Verbindung liegt vor, die Verbindung nur durch Zerstörung des Bauteils gelöst werden kann. Meist werden stoffschlüssige Verbindungen durch Sintern oder durch Verkleben von Materialien erreicht. Weitere bevorzugte Verfahren zum Herstellen einer stoffschlüssigen Verbindung sind: Löten, Schweissen und Infiltration.

In einer bevorzugten Ausgestaltung ist das Bauteil mit dem Befestigungselement, welches bevorzugt ein Cermet ist, durch mindestens eine Schweissverbindung verbunden. Bevorzugte und geeignete Schweissverfahren zum Herstellen einer solchen Schweissverbindung sind Laserschweissen oder Widerstandsschweissen.

In einer anderen bevorzugten Ausgestaltung ist das Bauteil mit dem Befestigungselement, welches bevorzugt ein Cermet ist, durch mindestens eine Lötverbindung verbunden.

Unter Infiltration wird das Bilden einer stoffschlüssigen Verbindung zwischen mindestens zwei Elementen verstanden, wobei mindestens eines der Element eine offenporig-poröse Oberfläche aufweist, die zum Herstellen des Stoffschlusses von einer flüssigen Phase benetzt und mindestens teilweise ausgefüllt wird. Die flüssige Phase erstarrt und stellt dabei einen Stoffschluss zwischen dem ersten und dem mindestens zweiten Element her. Die flüssige Phase kann aus verflüssigtem Material des zweiten Elements gebildet werden oder zum Herstellen des Stoffschlusses zusätzlich zu den mindestens zwei Elementen eingebracht werden Bevorzugt wird die stoffschlüssige Verbindung durch Sintern erreicht.

Beim Sintern gehen die stofflichen Eigenschaften des Grundkörpers fließend in die stofflichen Eigenschaften des Befestigungselements bzw. des elektrischen Leitungselements über. Es liegt keine scharfe Grenze zwischen den jeweiligen aneinandergrenzenden Bereichen vor. Vielmehr besteht ein Übergangsbereich in dem sich die Eigenschaften der beiden angrenzenden Bereiche mischen. Dieser Übergangsbereich wird auch als Mischbereich bezeichnet. In diesem Mischbereich liegen sowohl die Materialien des Grundkörpers als auch mindestens zum Teil die Materialien des Befestigungselementes bzw. des elektrischen Leitungselementes nebeneinander vor und bilden bevorzugt eine Vermischung der Materialien. Bevorzugt gehen die Materialien der beiden angrenzenden Bereiche Verbindungen auf atomarer oder molekularer Ebene ein.

In einer bevorzugten Ausgestaltung des Bauteils ist die Keramik des Grundkörpers ausgewählt aus der Gruppe bestehend aus Aluminiumoxid (Al₂O₃), Zirkoniumdioxid (ZrO₂) einem ein Aluminiumoxid enthaltendes Zirkoniumoxid (ATZ), einem ein Zirkoniumoxid enthaltendes Aluminiumoxid (ZTA), einem ein Yttrium enthaltendes Zirkoniumoxid (Y-TZP), Aluminiumnitrid (AlN), Titannitrid (TiN), Magnesiumoxid (MgO), einer Piezokeramik, Barium(Zr, Ti)oxid, Barium(Ce, Ti)oxid und Natrium-Kalium-Niobat oder einer Mischung aus mindestens zwei hiervon.

In einer bevorzugten Ausgestaltung des Bauteils ist das Metall des Befestigungselementes, oder das Metall des elektrischen Leitungselementes, oder beide Metalle ausgewählt aus der Gruppe bestehend Palladium (Pd), Platin (Pt), Gold (Au), Eisen (Fe), Edelstahl (AISI 304, AISI 316 L), Iridium (Ir), Niob (Nb), Molybdän (Mo), Wolfram (W), Titan (Ti), Kobalt (Co), Chrom (Cr), eine Kobalt-Chrom-Legierung, Tantal (Ta) und Zirkonium (Zr) oder einer Mischung aus mindestens zwei hiervon. Unter dieser Auswahl ist Platin bevorzugt.

Bevorzugt beinhaltet das Befestigungselement Platin in einem Bereich von 5 bis 30 Gew.-%, oder bevorzugt in einem Bereich von 10 bis 20 Gew.-%, oder bevorzugt in einem Bereich von 12 bis 18 Gew.-%, bezogen auf das Gesamtgewicht des Befestigungselements.

In einer bevorzugten Ausgestaltung des Bauteils ist der Metallgehalt des Befestigungselements größer als der Metallgehalt des elektrischen Leitungselementes. Bevorzugt beträgt der Metallgehalt des Befestigungselements zum Metallgehalt des elektrischen Leitungselements in einem Bereich von 1,1:1 bis 5:1, oder von 1,3:1 bis 3:1, oder von 1,5:1 bis 2:1, die Metallgehalte jeweils ausgedrückt in Gewichtsanteilen im Element.

In einer anderen bevorzugten Ausgestaltung des Bauteils ist der Metallgehalt des elektrischen Leitungselements größer als der Metallgehalt des Befestigungselementes. Bevorzugt beträgt der Metallgehalt des elektrischen Leitungselements zum Metallgehalt des Befestigungselements in einem Bereich von 1,1:1 bis 5:1, oder von 1,3:1 bis 3:1, oder von 1,5:1 bis 2:1, die Metallgehalte jeweils ausgedrückt in Gewichtsanteilen im Element.

In einer bevorzugten Ausgestaltung des Bauteils ist der Metallgehalt des elektrischen Leitungselements größer als der Metallgehalt des Befestigungselementes aufweist. Weiterhin bevorzugt beinhaltet das elektrische Leitungselement mehr Metall als das Befestigungselement in einem Bereich von 10 bis 500 %, oder bevorzugt in einem Bereich von 30 bis 300 %, oder bevorzugt in einem Bereich von 50 bis 200 %, die %-Angaben jeweils basierend auf das Metallmassedes elektrischen Leitungselements.

In einer bevorzugten Ausgestaltung des Bauteils weist das Bauteil mindestens ein elektrisches Leitungselement auf Bevorzugt weist das Bauteil mehr als ein elektrisches Leitungselement auf Bevorzugt weist das Bauteil mehr al 50, oder bevorzugt mehr als 100, oder bevorzugt mehr als 500 elektrische Leitungselemente auf Weiterhin bevorzugt weist das Bauteil elektrische Leitungselemente in einem Bereich von 2 bis 1000, oder bevorzugt in einem Bereich von 5 bis 500 oder bevorzugt in einem Bereich von 10 bis 200, oder bevorzugt in einem Bereich von 20 bis 100 auf.

In einer bevorzugten Ausgestaltung des Bauteils ist zumindest ein Teil einer der Bauteiloberflächen biokompatibel. Geeignete biokompatible Materialien werden weiter im Folgenden beschrieben.

Ein weiterer Gegenstand der Erfindung ist eine Vorrichtung, bevorzugt eine medizinische Vorrichtung, beinhaltend:
a. ein Gehäuse, das einen Innenbereich von einem Außenbereich mindestens zu einem Teil trennt, beinhaltend:
   i). eine Gehäusewand mit einer ersten Gehäuseoberfläche, die dem Innenbereich zugewandt ist und einer weiteren Gehäuseoberfläche, die dem Außenbereich des Gehäuses zugewandt ist;
   ii). mindestens eine Aussparung in der Gehäusewand;
b. ein erfindungsgemäßes Bauteil wie im ersten Gegenstand der Erfindung oder in einer der bevorzugten Ausgestaltungen dazu beschrieben.

Die Vorrichtung kann jede Vorrichtung sein, die der Fachmann zusammen mit einem Bauteil verwenden würde. Bevorzugt ist die Vorrichtung eine medizinische Vorrichtung. Weiterhin bevorzugt ist die Vorrichtung dazu geeignet mit dem Körper eines Benutzers in Kontakt gebracht zu werden oder bevorzugt in den Körper des Benutzers eingebracht zu werden. Bevorzugt ist die medizinische Vorrichtung ausgewählt aus der Gruppe bestehend aus einer therapeutischen Vorrichtung und einer diagnostischen Vorrichtung oder einer Kombination aus beiden.

Das Gehäuse kann jedes Gehäuse sein, das der Fachmann zur Verbindung mit dem Bauteil einsetzen würde. Das Gehäuse beinhaltet bevorzugt mindestens eine Keramik, ein Glas, einen Kunststoff, oder einMetall, insbesondere ein biokompatibles Metall. Das Metall ist bevorzugt ausgewählt aus der Gruppe bestehend aus Platin, Palladium, Titan, Tantal, Niob, Stahl oder eine Mischung aus mindestens zwei hiervon. Das Gehäuse beinhaltet das Metall bevorzugt in einem Bereich von 10 bis 60 Gew.-%, oder bevorzugt in einem Bereich von 20 bis 50 Gew.-%, oder bevorzugt in einem Bereich von 30 bis 40 Gew.-%, bezogen auf das Gesamtgewicht des Gehäuses.

Das Gehäuse kann jede Form aufweisen, die der Fachmann für die Vorrichtung auswählen würde. Bevorzugt weist das Gehäuse wenig bis keine Ecken und Kanten auf. Bevorzugt ist die Form des Gehäuses ausgewählt aus der Gruppe bestehend aus rund, oval, zylindrisch, rechteckig oder einer Kombination aus mindestens zwei hiervon. Das Volumen der Vorrichtung liegt bevorzugt in einem Bereich von 1 bis 1000 cm³, oder bevorzugt in einem Bereich von 2 bis 500 cm³, oder bevorzugt in einem Bereich von 5 bis 100 cm³. Weiterhin bevorzugt umschließt das Gehäuse den Innenbereich zusammen mit dem Bauteil vollständig.

Die Aussparung in der Gehäusewand ist bevorzugt so ausgewählt, dass sie mindestens einen Teil des Bauteils in sich aufnehmen kann. Bevorzugt ist die Aussparung so ausgestaltet, dass sie das Bauteil bündig umschließt. Weiterhin bevorzugt wird das Bauteil mit der Gehäusewand des Gehäuses der Vorrichtung, im Bereich der Aussparung, verbunden. Bevorzugt findet die Verbindung der Gehäusewand mit dem Bauteil über das mindestens eine Befestigungselement statt. Alternativ oder zusätzlich können das Bauteil oder das Gehäuse weitere Elemente beinhalten, die zur hermetischen Abdichtung des Bauteils mit dem Gehäuse dienen. So kann beispielsweise ein Ring aus einem elastischen Material, wie Kautschuk, in die Aussparung zwischen Gehäuse und Bauteil angebracht sein, der die Vorrichtung hermetisch abschließt. Die Verbindung des Gehäuses mit dem Bauteil über die Befestigungselemente dient dann lediglich zur Fixierung des Bauteils in der Aussparung des Gehäuses.

Weiterhin bevorzugt ist die Verbindung zwischen dem Bauteil und dem Gehäuse hermetisch dicht. In einer bevorzugten Ausgestaltung der Vorrichtung weist das erste Ende des mindestens einen elektrischen Leitungselements zum Innenbereich und das weitere Ende des mindestens einen elektrischen Leitungselements zum Außenbereich des von dem Gehäuse umschlossenen Innenbereiches. Auf diese Weise kann der hermetisch abgeschlossene Innenbereich des Gehäuses der Vorrichtung elektrisch mit dem Außenbereich verbunden werden, ohne dass ein Flüssigkeits- oder Gasaustausch zwischen Innenberiech und Außenbereich möglich ist.

Im Rahmen der Erfindung bedeutet der Begriff "hermetisch dicht", dass der zu prüfende Gegenstand gemäß der in MIL-STD-883G, Method 1014.13 (Stand: 26. Februar 2010), Absatz 3.1 definierten Methode A₁ eine maximal zulässige Helium-Leckrate von 10⁻⁷ atm*cm³/sec oder weniger aufweist. In einer besonders vorteilhaften Ausführung beträgt die Helium-Leckrate weniger als 1 x 10⁻⁸ atm*cm³/sec, insbesondere weniger als 1 x 10⁻⁹ atm*cm³/sec. Der zu prüfende Gegenstand kann zum Beispiel ein Bauteil, oder auch ein Bauteil mit einer hermetisch dichten Verbindung von mehreren Elementen sein.

In einer bevorzugten Ausgestaltung der Vorrichtung beinhaltet die Gehäusewand mindestens 30 Gew.-% Titan, bezogen auf das Gesamtgewicht des Gehäuses. Weiterhin bevorzugt beinhaltet die Gehäusewand Titan in einem Bereich von 30 bis 100 Gew.-%, oder bevorzugt in einem Bereich von 40 bis 90 Gew.-%, oder bevorzugt in einem Bereich von 50 bis 85 Gew.-%, bezogen auf das Gesamtgewicht des Gehäuses. In einer bevorzugten Ausgestaltung der Vorrichtung beinhaltet die Gehäusewand eine äußere Schicht, die mindestens 30 Gew.-%, bezogen auf die äußere Schicht der Gehäusewand, Titan beinhaltet. Das Gehäuse kann weitere Komponenten ausgewählt aus der Gruppe bestehend aus einem Polymer, einer Keramik, einem Metall oder einer Mischung oder Kombination hieraus beinhalten. Das Polymer ist bevorzugt ausgewählt aus der Gruppe bestehend aus einem Polycarbonat, einem Polyethylen, einem Polyester, einem Polyether, einem Polyvinylcarbonat, einem Polypropylen, einem Polystyrol, einem Polyetheretherketon, einem Polyamin, einem Polyamid, einem Polyimin, einem Polyimid, einem Polyterephthalat oder einer Mischung aus mindestens zwei hieraus. Die Keramik kann jede Keramik sein, wie sie für das Bauteil beschrieben wurde. Das Metall kann ausgewählt sein aus der Gruppe bestehend aus Palladium, Stahl, Nickel, Chrom, Tantal, Wolfram, Zirkonium, Iridium, Titan, Niob, oder einer Mischung aus mindestens zwei hieraus.

In einer bevorzugten Ausgestaltung der Vorrichtung ist zumindest ein Teil des Bauteils, der dem Außenbereich zugewandt ist und zumindest der Teil der Gehäuseoberfläche, die dem Außenbereich zugewandt ist, biokompatibel. Dies ist insbesondere bevorzugt, wenn die Vorrichtung für die Implantation in einen lebenden Körper, wie beispielsweise den eines Menschen oder Tieres vorgesehen ist. Die Biokompatibilität wird ermittelt gemäß der Norm ISO 10993-4:2002.

In der Regel kommen die dem Innenbereich der Vorrichtung zugewandten Oberflächen und die Außenflächen des Bauteils nach Implantieren der erfindungsgemäßen Vorrichtung in einen lebenden Körper mit dessen Körperflüssigkeit in Kontakt. Die Biokompatibilität der mit Körperflüssigkeit in Berührung kommenden Oberflächen trägt dazu bei, dass der Körper beim Kontakt mit diesen Oberflächen keinen Schaden nimmt.

Ein weiterer Gegenstand der Erfindung ist ein Verfahren zur Herstellung eines Bauteils beinhaltend mindestens folgende Schritte:
I. Bereitstellen einer ersten Masse zur Formung eines ersten Teilbereiches;
II. Bereitstellen einer weiteren Masse zur Formung mindestens eines weiteren Teilbereiches;
III. Bereitstellen einer dritten Masse zur Formung eines dritten Teilbereiches;
IV. Bilden eines Bauteilvorläufers, wobei ein erster Vorläufer für einen Grundkörper aus dem ersten Teilbereich gebildet wird, wobei ein weiterer Vorläufer für ein elektrisches Leitungselement aus dem mindestens einen weiteren Teilbereich gebildet wird und wobei ein dritter Vorläufer für mindestens ein Befestigungselement aus dem dritten Teilbereich gebildet wird;
V. Behandeln des Bauteilvorläufers bei einer Temperatur in einem Bereich von 500 bis 2300 °C, bevorzugt in einem Bereich von 600 bis 2000 °C oder bevorzugt in einem Bereich von 1000 bis 1800 °C;
wobei sich der weitere Vorläufer durch den gesamten ersten Vorläufers hindurch erstreckt,
wobei mindestens ein Teil der dritten Masse von einem Teil der ersten Masse umgeben ist.

Die erste Masse weist bevorzugt mindestens eine erste Komponente auf Weiterhin kann die erste Masse mindestens eine weitere Komponente aufweisen. Die erste Komponente kann jede Substanz beinhalten, die dazu beiträgt, aus dem Vorläufer für einen Bauteilvorläufer durch Erhitzen ein Sinterprodukt zu erhalten.

Die erste Komponente ist beispielsweise ein Ausgangsmaterial für eine sinterfähige Substanz. Bevorzugt beinhaltet die erste Komponente ein Ausgangsmaterial für eine Keramik ausgewählt aus der Gruppe bestehend aus einer Oxidkeramik, einer Silikatkeramik, einer Nichtoxid-Keramik oder einer Mischung aus mindestens zwei davon. Details zu den verschiedenen Keramiken wurde bereits für das erfindungsgemäße Bauteil beschrieben. Zusammensetzung, Form und Struktur wie für das Bauteil beschrieben, sind hier für das Produkt des Verfahrens zur Herstellung eines Bauteils ebenfalls anzuwenden.

Der Vorläufer für den Grundkörper, auch Grundkörpervorläufer genannt, kann darüber hinaus weitere Komponenten beinhalten. So kann der Grundkörpervorläufer als weitere Komponente jede Substanz beinhalten, die der Fachmann auswählen würde, um ein Mischen der verschiedenen Komponenten des Grundkörpervorläufers zu erleichtern. Bevorzugt beinhaltet die weitere Komponente ein Bindemittel. Beispiele für Bindemittel sind Methylcellulose, 2,2,4-Trimethylpentan-1,3-diol-monoisobutyrat oder eine Mischung hieraus. Die Methylcellulose ist bevorzugt ausgewählt aus der Gruppe bestehend aus Hydroxypropylmethylcellulose (HPMC), Hydroxyethylmethylcellulose (HEMC), Ethylmethylcellulose (EMC) oder einer Mischung hieraus.

Als weitere Bindemittel können beispielsweise thermoplastische oder duroplastische Polymere oder Wachse verwendet werden. Dabei können diese allein oder als Gemische von Bindemitteln mehrerer solcher Komponenten eingesetzt werden. Das thermoplastische Polymer kann ausgewählt sein aus der Gruppe bestehend aus Acrylnitril-Butadien-Styrol (ABS), Polyamide (PA), Polylactat (PLA), Polymethylmethacrylat (PMMA), Polycarbonat (PC), Polyethylenterephthalat (PET), Polyethylen (PE), Polypropylen (PP), Polystyrol (PS), Polyetheretherketon (PEEK) und Polyvinylchlorid (PVC), Polyvinylbutyral (PVB) oder einer Mischung von mindestens zwei davon. Das duroplastische Polymer kann ausgewählt sein aus der Gruppe bestehend aus einem Aminoplasten, einem Epoxidharz, einem Phenolharz, einem Polyester-Harz oder einer Mischung aus mindestens zwei davon. Wachse sind Kohlenwasserstoffverbindungen, die bei Raumtemperatur knetbar und/oder fest sind und oberhalb 40 °C ohne Zersetzung schmelzen. Hierunter können sich auch Polyester, Paraffine, Polyethylene oder Copolymere aus mindestens zwei daraus befinden. Der Vorläufer für den Grundkörper beinhaltet das Bindemittel bevorzugt in einem Bereich von 1 bis 30 Gew.-%, oder bevorzugt in einem Bereich von 3 bis 25 Gew.-%, oder bevorzugt in einem Bereich von 5 bis 20 Gew.-%, bezogen auf die Gesamtgewicht des Vorläufers für den Grundkörper. Nach Behandlung des Bauteilvorläufers weist der dabei entstehende Grundkörper die Anteile an Keramik und Metall auf, wie sie bereits für den Grundkörper beschrieben wurden. Die Summe aller Bestandteile des Vorläufers für den Grundkörper ergibt stets 100 Gew.-%.

Der weitere Vorläufer für das elektrische Leitungselement beinhaltet bevorzugt eine Keramik und ein Metall, wie sie zuvor für das elektrische Leitungselement beschrieben wurden. Darüber hinaus kann der weitere Vorläufer für das elektrische Leitungselement ein Bindemittel beinhalten, wie es für den ersten Vorläufer für den Grundkörper beschrieben wurde. Der weitere Vorläufer für das elektrische Leitungselement beinhaltet das Bindemittel bevorzugt in einem Bereich von 1 bis 30 Gew.-%, oder bevorzugt in einem Bereich von 3 bis 25 Gew.-%, oder bevorzugt in einem Bereich von 5 bis 20 Gew.-%, bezogen auf die Gesamtgewicht des Vorläufers für das elektrische Leitungselement. Die weiteren Komponenten, wie die Keramik und das Metall liegen im gleichen Verhältnis zueinander vor, wie für das elektrische Leitungselement bereits beschrieben. Ihr Gesamtanteil reduziert sich im Vorläufer für das elektrische Leitungselement entsprechend des Einsatzes des Bindemittels in dem Vorläufer für das elektrische Leitungselement. Nach Behandlung des Bauteilvorläufers weist das dabei entstehende elektrische Leitungselement die Anteile an Keramik und Metall auf, wie sie bereits für das elektrische Leitungselement beschrieben wurden. Die Summe aller Bestandteile des Vorläufers für das elektrische Leitungselement ergibt stets 100 Gew.-%.

Der dritte Vorläufer für das Befestigungselement beinhaltet bevorzugt eine Keramik und ein Metall, wie sie zuvor für das Befestigungselement beschrieben wurden. Darüber hinaus kann der dritte Vorläufer für das Befestigungselement ein Bindemittel beinhalten, wie es für den ersten Vorläufer für den Grundkörper beschrieben wurde. Der dritte Vorläufer für das Befestigungselement beinhaltet das Bindemittel bevorzugt in einem Bereich von 1 bis 30 Gew.-%, oder bevorzugt in einem Bereich von 3 bis 25 Gew.-%, oder bevorzugt in einem Bereich von 5 bis 20 Gew.-%, bezogen auf die Gesamtgewicht des Vorläufers für das Befestigungselement. Die weiteren Komponenten, wie die Keramik und das Metall liegen im gleichen Verhältnis zueinander vor, wie für das Befestigungselement bereits beschrieben. Ihr Gesamtanteil reduziert sich im Vorläufer für das Befestigungselement entsprechend des Einsatzes des Bindemittels in dem Vorläufer für das Befestigungselement. Nach Behandlung des Bauteilvorläufers weist das dabei entstehende elektrische Leitungselement die Anteile an Keramik und Metall auf, wie sie bereits für das elektrische Leitungselement beschrieben wurden. Die Summe aller Bestandteile des Vorläufers für das elektrische Leitungselement ergibt stets 100 Gew.-%.

Neben einem Bindemittel kann die erste, die weitere oder die dritte Masse auch mindestens ein Lösungsmittel beinhalten. Als Lösungsmittel kommen beispielsweise Wasser, einem Alkohol, einem Amin, einer Säure, einer Lauge oder eine Mischung aus mindestens zwei hiervon. Der Alkohol kann ausgewählt sein aus der Gruppe bestehend aus Methanol, Ethanol, 1-Propanol, 2- Propanol, 1-Butanol, 2-Butanol, 2-Methyl-1-propanol und 2-Methyl-2-propanol, Methoxypropanol, Ethoxypropanol, Methoxyethanol, Ethoxyethanol, 4-Hydroxymethyl-1,3-dioxalon vorzugsweise Methanol, Ethanol, Propanol, Butanol oder einer Mischung aus mindestens zwei hiervon.

Das Amin kann ausgewählt sein aus der Gruppe bestehend aus Ammoniak, Methylamin, Ethylamin, n-Propylamin, i-Propylamin, n-Butylamin, Dimethylamin, Diethylamin, Di-n-propylamin, Di-n-butylamin, Pyrrolidin, Piperidin, Piperazin, N-Methyl-piperazin, N-Ethylpiperazin, Morpholin, Ethylendiamin, 1,2-Propylendiamin, 1,3-Propylendiamin, Di-(2-cyanoethyl)amin, Di-(2-aminoethyl)amin, Tri-(2-aminoethyl)-amin, Ethanolamin, Diethanolamin, Triethanolamin, Propanolamin, Dipropanolamin und Tripropanolamin oder einer Mischung aus mindestens zwei hiervon.

Die Säure kann ausgewählt sein aus der Gruppe bestehend aus Salzsäure, Salpetersäure, Schwefelsäure, Phosphorsäure oder einer Mischung aus mindestens zwei hiervon.

Die Lauge kann ausgewählt sein aus der Gruppe bestehend aus Natriumhydroxid, Kaliumhydroxid, Lithiumhydroxid, Ammoniumhydroxid oder einer Mischung aus mindestens zwei hiervon.

Die erste, die weitere oder die dritte Masse beinhalten das Lösungsmittel bevorzugt in einem Bereiche von 0,1 bis 5 Gew.-%, bevorzugt in einem Bereiche von 0,2 bis 3 Gew.-%, bevorzugt in einem Bereiche von 0,5 bis 2 Gew.-%, jeweils bezogen auf das Gesamtgewicht der ersten, weiteren oder dritten Masse.

In einer bevorzugten Ausgestaltung des Verfahrens beinhaltet Schritt IV. einen Formgebungsprozess, bevorzugt ausgewählt aus der Gruppe bestehend aus einem lithographischen Prozess, einem Spritzgießen, einem Zerspanen, einem Extrudieren oder einer Kombination von mindestens zwei hiervon.

In einem lithographischen Prozess werden verschiedene Schichten eines oder mehrerer Materialien nacheinander in eine Form gebracht. Der lithographische Prozess entspricht bevorzugt einem schichtweisen Siebdruckverfahren. Beim Siebdruckverfahren wird ein Sieb, bestehend aus einem möglichst formstabilen Material, wie Holz; Metall, bevorzugt Stahl; einer Keramik oder einem Kunststoff mit einer ausgewählten Maschenweite auf das zu überlagernde oder über dem zu überlagernden Objekt, angeordnet. Auf dieses Sieb wird über eine Düse oder aus einem Behälter die zum Aufbringen oder Überlagern verwendete Druckmasse, beispielsweise in Form eine Paste oder eines Pulvers, aufgebracht und mit einer Rakel durch die Maschen des Siebs gedrückt. Dabei kann aufgrund eines Musters in dem Sieb an unterschiedlichen Stellen unterschiedlich viel, zum Aufbringen oder Überlagern verwendete Druckmasse aufgebracht werden. So kann durch die Geometrie und Anordnung der Maschen entweder ein gleichmäßiger Film der zum Überlagern verwendeten Druckmasse aufgebracht werden oder Bereiche mit keiner oder wenig zum Aufbringen verwendeten Druckmasse mit Bereichen mit viel zum Aufbringen verwendeten Druckmasse abwechseln. Bevorzugt wird ein gleichmäßiger Film der zum Überlagern verwendeten Druckmasse auf die Oberfläche übertragen. Die Siebmaschen können auch durch entsprechend aufgebrachte Materialien (Kopierschichten, Siebdruckschablonen) teilweise geschlossen sein, so dass die Druckmasse nur in definierten Bereichen mit offenen Maschen auf die zu beschichtende Oberfläche übertragen wird, um so beispielsweise eine definierte Struktur wie ein Muster zu erhalten. Weiterhin können statt Sieben auch dünne Filme mit definierten Öffnungen (Stencil) zum Übertragen der Druckmasse verwendet werden. Durch Wiederholung dieses Vorgangs mit ein und demselben Material oder auch unterschiedlichen Materialien können 3-D Strukturen, wie der erfindungsgemäße Bauteilvorläufer oder Teile davon, erhalten werden.

Das Spritzgießen, oder auch Spritzgussverfahren genannt, ist ein Formungsprozess für mindestens ein Material, um einen geformten Festkörper zu erhalten. Dem Fachmann sind unterschiedliche Spritzgießverfahren sowie beim Spritzgießen verwendeten Werkzeuge und Bedingungen aus dem Stand der Technik bekannt. Das Spritzgießen kann ausgewählt sein aus der Gruppe bestehend aus einem Mehrkomponenten-Spritzgießen, einem Pulverspritzgießen, einem Spritzprägen, einem Extrusionsspritzgießen, einem Unterdruckspritzgießen oder einer Kombination aus mindestens zwei hiervon.

Das Zerspanen kann mit jedem anderen Formgebungsprozess kombiniert werden. Beim Zerspanen wird ein massiver Körper durch Einsatz von Zerspanungshilfsmitteln, wie einem Bohrer strukturiert. Bei dem Strukturieren wird ein Teil des Materials abgetragen. Hierdurch können massive Körper beispielsweise zu Hohlkörpern geformt werden. Beispielsweise kann durch Zerspanen in den Bauteilvorläufer ein Hohlraum geformt werden Zusätzlich zum Zerspanen kann im Anschluss ein Polieren stattfinden.

In einer bevorzugten Ausgestaltung des Verfahrens erfolgt in Schritt IV. ein Behandeln des Bauteilvorläufers bei einer Temperatur in einem Bereich von 120 bis 500 °C, oder bevorzugt bei einer Temperatur in einem Bereich von 150 bis 450 °C, oder bevorzugt bei einer Temperatur in einem Bereich von 200 bis 400 °C vor dem Behandeln in Schritt V.. Das Behandeln des Bauteilvorläufers in Schritt IV. bzw. in Schritt V., das auch Erhitzen genannt wird, kann auf jede Art auf den Vorläufer erfolgen, die der Fachmann für diesen Zweck auswählen würde. Das Erhitzen ist bevorzugt ein Erhitzen mittels einer Methode ausgewählt aus der Gruppe bestehend aus einem Bestrahlen, einem Erhitzen in einem Ofen, einem Erhitzen mit heißem Gas oder einer Kombination aus mindestens zwei hieraus. Die Bestrahlung kann beispielsweise mittel IR-Strahlung, Laserstrahlung, UV-Strahlung oder einer Kombination hieraus erfolgen. Das Erhitzen in einem Ofen, wie beispielsweise einem Heißluftofen, kann beispielsweise diskontinuierlich oder kontinuierlich erfolgen. Das Erhitzen mit einem heißen Gas kann beispielsweise durch Überleiten eines heißen Gasstroms wie Luft, Stickstoff, Sauerstoff oder einem Gemisch hieraus über die aufgebrachte Zusammensetzung erfolgen. Die Dauer des Erhitzens in Schritt IV. oder in Schritt V. findet jeweils bevorzugt in einem Bereich von 0,3 bis 10 h, oder bevorzugt in einem Bereich von 0,4 bis 5 h, oder bevorzugt in einem Bereich von 0,5 bis 3h statt.

In einer bevorzugten Ausgestaltung der Verfahrens ist die erste Masse zur Formung des ersten Teilbereiches und Bildung des ersten Vorläufers für den Grundkörper so ausgestaltet, dass sie im Behandlungsschritt V. um mindestens 1 %, oder bevorzugt um mindestens 2 %, oder bevorzugt um mindestens 3 %, bezogen auf das Volumen des ersten Vorläufers mehr schrumpft als die weitere Masse zur Formung des weiteren Vorläufers für das Leitungselement, bezogen auf das Volumen des weiteren Vorläufers. Weiterhin bevorzugt ist die dritte Masse zur Formung des dritten Teilbereiches und Bildung des Befestigungselements so ausgestaltet, dass sie im Behandlungsschritt V. um höchstens 5 %, oder bevorzugt um höchstens 3 %, oder bevorzugt um höchstens 2 %, bezogen auf das Volumen des ersten Vorläufers mehr schrumpft als die erster Masse zur Formung des ersten Vorläufers für den Grundkörper, bezogen auf das Volumen des ersten Vorläufers.

Ein weiterer Gegenstand der Erfindung ist ein Bauteil erhältlich nach dem zuvor beschriebenen Verfahren.

Ein weiterer Gegenstand der Erfindung ist eine Vorrichtung beinhaltend mindestens ein erfindungsgemäßes Bauteil. Bei der Vorrichtung kann es sich beispielsweise um eine medizinische Vorrichtung handeln, wie sie zuvor beschrieben wurde.

In einer bevorzugten Ausgestaltung der Vorrichtung beinhaltet die Vorrichtung mindestens ein Gehäuse mit mindestens einer Gehäuseöffnung und mindestens einem erfindungsgemäßen Bauteil, wobei das Bauteil über das mindestens eine Befestigungselement mit dem Gehäuse verbunden ist, wobei durch das Bauteil mindestens eine elektrische Verbindung zwischen mindestens einem Innenbereich des Gehäuses und mindestens einem Außenbereich hergestellt ist. Wie bereits zuvor beschrieben, wird durch die Verbindung des Bauteils mit dem Gehäuse eine hermetische Abdichtung des Innenbereiches gegenüber dem Außenbereich erreicht. Die Materialien des Gehäuses und des Bauteils, aber auch die Art der Anordnung sowie der Befestigung des Bauteils in der Vorrichtung kann auf die gleiche Weise erfolgen, wie bereits für die Vorrichtung oben beschrieben.

### Messmethoden

### 1. Bestimmung der Biokompatibilität:

Die Biokompatibilität wird gemäß der Norm nach 10993-4:2002 bestimmt.

### 2. Bestimmung der Hermetischen Verbindung:

Lecktests werden mit Heliumlecktestern und/oder Massenspektrometern durchgeführt werden. Das Standardmessverfahren ist im Standard Mil-STD-883G Method 1014.13 (Stand 26. Februar 2010) spezifiziert. Die maximal zulässige Helium-Leckrate wird dabei abhängig vom internen Volumen der zu prüfenden Vorrichtung festgelegt. Nach den in MIL-STD-883G, Method 1014.13, Absatz 3.1, Methode A₁ beträgt die maximal zulässige Helium-Leckrate für die erfindungsgemäßen Vorrichtungen mit Gehäuse 10 ⁻⁷ atm*cm³/sec. Das bedeutet, dass die zu prüfende Vorrichtung (beispielsweise das Gehäuse mit Bauteil und/oder die Vorrichtung) eine Helium-Leckrate 1 x 10⁻⁷ atm*cm³/sec oder weniger aufweist.

### Beispiele

### Beispiel 1 für eine Zusammensetzung der ersten Masse:

Die erste Masse enthält 70 Gew.-% Aluminiumoxid (Al₂O₃) der Firma CeramTech GmbH mit einer Korngröße von D₉₀ = 2 µm sowie 30 Gew.-% eines Bindemittels METAWAX P-50 erhältlich bei der Firma Zschimmer & Schwarz GmbH & Co.KG.

### Beispiel 2 für eine Zusammensetzung der weiteren Masse:

Die weitere Masse enthält eine Mischung aus 70 Gew.-% Platinpulver der Firma Heraeus Precious Metals GmbH & Co.KG mit einer Korngröße D₅₀ = 2 µm sowie 15 Gew.-% Aluminiumoxid (Al₂O₃) erhältlich bei der Firma CeramTech GmbH, sowie 15 Gew.-% des Bindemittels METAWAX P-50 erhältlich bei der Firma Zschimmer & Schwarz GmbH & Co.KG.

### Beispiel 3 für eine Zusammensetzung der dritten Masse:

Die dritte Masse enthält eine Mischung aus 75 Gew.-% Platinpulver der Firma Heraeus Precious Metals GmbH & Co.KG mit einer Korngröße D₅₀ = 5 µm sowie 12,5 Gew.-% Aluminiumoxid (Al₂O₃) erhältlich bei der Firma CeramTech GmbH, sowie 12,5 Gew.-% des Bindemittels METAWAX P-50 erhältlich bei der Firma Zschimmer & Schwarz GmbH & Co.KG.

### Beispiel 4 für eine Zusammensetzung des Grundkörpers

Der Grundkörper enthält 100 Gew.-% Aluminiumoxid (Al₂O₃) der Firma CeramTech GmbH.

### Beispiel 5 für eine Zusammensetzung des elektrischen Leitungselements:

Das elektrische Leitungselement enthält 85 Gew.-% Platinpulver der Firma Heraeus Precious Metals GmbH & Co.KG und 15 Gew.-% Aluminiumoxid (Al₂O₃) der Firma CeramTech GmbH.

### Beispiel 6 für eine Zusammensetzung des Befestigungselements:

Das Befestigungselement enthält 80 Gew.-% Platinpulver der Firma Heraeus Precious Metals GmbH & Co.KG und 20 Gew.-% Aluminiumoxid (Al₂O₃) der Firma CeramTech GmbH.

Aus der Zusammensetzung nach Beispiel 2 wird ein Cermetgrünkörper eines Leitungselements erstellt. Hierzu wird die Zusammensetzung nach Beispiel 2 aufgeschmolzen und in eine Negativform eingefüllt. Die Negativform wird entfernt nach dem sich die Zusammensetzung nach Beispiel 2 wieder verfestigt hat.

Aus der Zusammensetzung nach Beispiel 1 wird ein Grundgrünkörper eines als Isolator fungierenden Grundkörpers erstellt. Hierzu wird die Zusammensetzung nach Beispiel 1 aufgeschmolzen und in eine Negativform eingefüllt, wobei die Negativform ein sich mit den Dimensionen des Cermetgrünkörpers deckenden Loch im Inneren und eine umlaufende Feder zum Ausbilden einer Nut in der äußeren Kontaktfläche des Grundgrünkörpers aufweist. Die Negativform wird entfernt nach dem sich die Zusammensetzung nach Beispiel 1 wieder verfestigt hat.

Aus der Zusammensetzung nach Beispiel 3 wird ein Befestigungselementgrünkörper eines als Umfassung des Grundkörpers fungierenden Befestigungselements erstellt. Hierzu wird die Zusammensetzung nach Beispiel 3 aufgeschmolzen und in eine Negativform eingefüllt, wobei die Negativform eine sich mit der Nut der Kontaktfläche des Grundgrünkörper deckende Dimensionierung aufweist. Die Negativform wird entfernt nach dem sich die Zusammensetzung nach Beispiel 3 wieder verfestigt hat.

Der Cermetgrünkörper wird in die Öffnung des Grundgrünkörpers gesetzt. Der den Cermetgrünkörper enthaltende Grundgrünkörper wird über die Kontaktfläche mit dem Befestigungselementgrünkörper über Nut und Feder verbunden. Das so erhaltene Bauteil wird in einem Kammerofen der Firma Nabertherm GmbH bei 1600°C über 2 Stunden 15 Minuten gesintert. Das so erhaltene Sintergut wurde in dem geschlossenen Ofen durch Abschalten des Ofens auf Raumtemperatur abgekühlt und entnommen. Die Zusammensetzungen der einzelnen Komponenten des so erhaltenen Bauteils sind in den Beispielen 5 bis 6 angegeben.

### Figuren

Im Folgenden wird in
- Figur 1a: eine schematische Darstellung eines würfelförmigen, erfindungsgemäßen Bauteils mit mehreren Befestigungselementen;
- Figur 1b: eine schematische Darstellung eines würfelförmigen, erfindungsgemäßen Bauteils mit einem umlaufenden Befestigungselement;
- Figur 2a: eine schematische Darstellung eines zylindrischen, erfindungsgemäßen Bauteils mit mehreren Befestigungselementen;
- Figur 2b: eine schematische Darstellung eines zylindrischen, erfindungsgemäßen Bauteils mit einem umlaufenden Befestigungselement;
- Figur 3: eine erfindungsgemäße Vorrichtung mit einem erfindungsgemäßen Bauteil;
- Figur 4: ein Schema eines Verfahrens zur Herstellung eines erfindungsgemäßen Bauteils;
gezeigt.

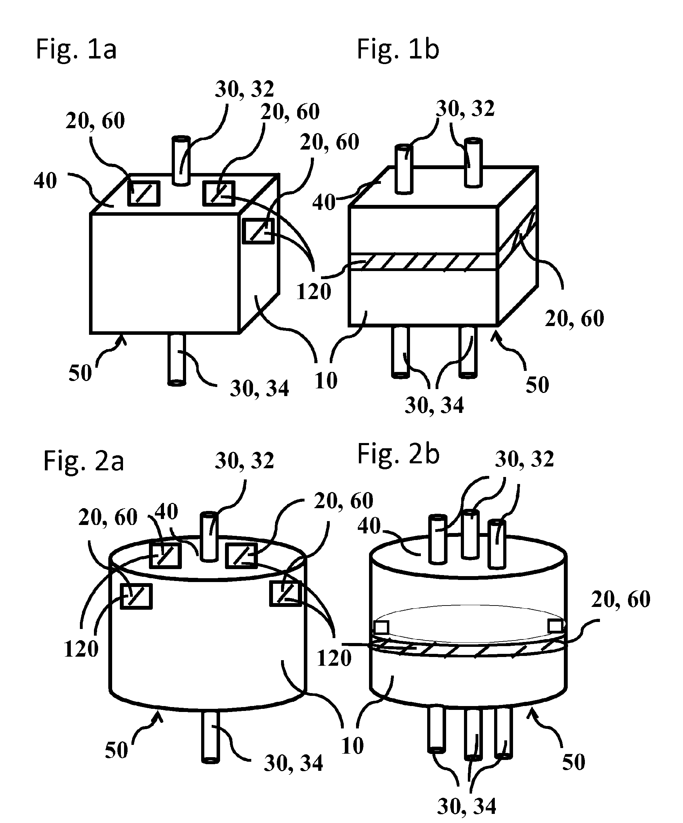

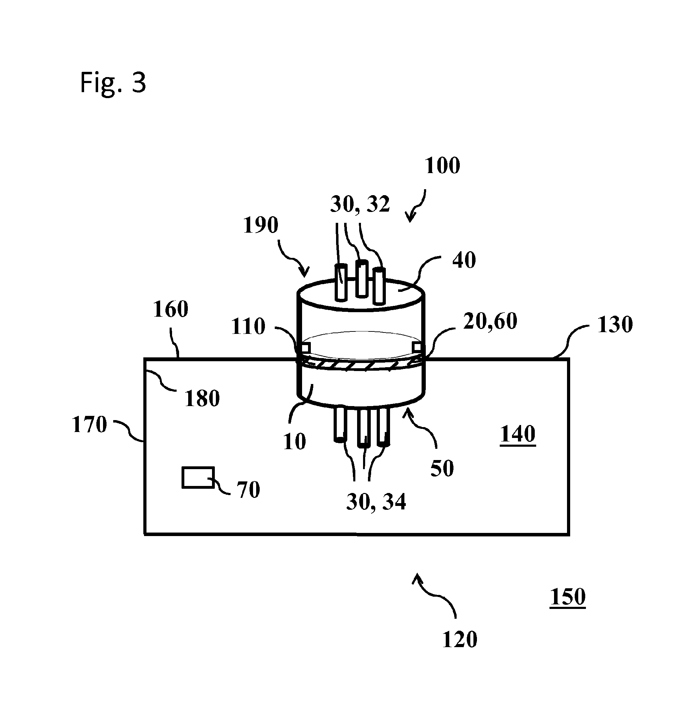

In Figur 1a ist ein würfelförmiges Bauteil 100 perspektivisch gezeigt. Das Bauteil 100 weist eine erste Bauteiloberfläche 40 und eine weitere Bauteiloberfläche 50 auf. Das Bauteil 100 beinhaltet einen Grundkörper 10, durch dessen Inneres ein elektrisches Leitungselement 30 geführt ist. Das elektrische Leitungselement 30 weist ein erstes Ende 32 und ein weiteres Ende 34 auf. Das erste Ende 32 des elektrischen Leitungselements 30 ragt aus der ersten Bauteiloberfläche 40 heraus, während das weitere Ende 34 des elektrischen Leitungselements 30 aus der weiteren Bauteiloberfläche 50 herausragt. Das elektrische Leitungselement 30 verbindet dadurch die erste Bauteiloberfläche 40 elektrisch mit der weiteren Bauteiloberfläche 50 durch den Grundkörper 10 hindurch. Weiterhin sind an verschiedenen Stellen auf der Oberfläche des Grundkörpers 10 Befestigungselement 20 eingebracht. In Figur 1a sind exemplarisch zwei Befestigungselemente 20 auf der ersten Bauteiloberfläche 40 angeordnet und ein weiteres Befestigungselement 20 an der Seite des Bauteils 100 zwischen erster Bauteiloberfläche 40 und zweiter Bauteiloberfläche 50. Die Befestigungselemente 20 weisen alle einer Kontaktfläche 60 auf, mit der sie an beispielsweise ein Gehäuse einer Vorrichtung 120, wie in Figur 3 gezeigt kontaktiert werden können. Die Kontaktfläche 60 ist der Teil der Oberfläche 110 des Befestigungselements 20, die nicht von dem Grundkörper 10 umgeben ist, sondern zu einer Kontaktierung mit beispielsweise einem Gehäuse einer Vorrichtung frei zugänglich ist. Es können sich auch Befestigungselemente 20 auf der weiteren Bauteiloberfläche 50 befinden (hier nicht sichtbar). Eine weitere Ausführungsform mit einer Vielzahl von Befestigungselementen 20 wäre auch zusammen mit einer Mehrzahl von elektrischen Leitungselementen 30 denkbar. Die elektrischen Leitungselemente 30 verbinden dabei immer die erste Bauteiloberfläche 40 mit der weiteren Bauteiloberfläche 50 durch den Grundkörper 10 hindurch. Die Anordnung, Zahl und Form der Befestigungselemente 20 ist frei wählbar.

Figur 1b zeigt ein Bauteil 100 mit der gleichen Form, wie das Bauteil 100 aus Figur 1a, mit dem Unterschied, dass es nur ein anstatt mehrerer Befestigungselemente 20 zum Teil in den Grundkörper 10 eingelassen gibt. Ein weiterer Unterschied zu dem Bauteil 100 aus Figur 1a ist die Anordnung eines zweiten elektrischen Leitungselements 30. Beide elektrischen Leitungselemente weisen ein erstes Ende 32 auf, das aus der ersten Bauteiloberfläche 40 ragt auf und ein weiteres Ende 34, das aus der weiteren Bauteiloberfläche 50 ragt. Das Befestigungselement 20 weist ebenfalls eine Kontaktfläche 60 auf. Die Kontaktfläche 60 ist läuft in dieser Ausführungsform komplett um den Grundkörper 10 des Bauteils 100 herum. Die Kontaktfläche 60 bildet in diesem Beispiel einen Ring um den Grundkörper 10 herum.

In Figur 2a ist ein Bauteil 100 mit einem zylindrischen Grundkörper 10 gezeigt. Hier sind, wie in Figur 1a ebenfalls mehrere Befestigungselemente 20 in den Grundkörper 10 mindestens zu einem Teil eingebracht und von diesem mindestens zu einem Teil umgeben. Die Kontaktfläche 60 ist jedoch, wie auch in den Beispielen aus Figur 1a und 1b frei zugänglich. Die Befestigungselemente 20 können sich, wie in Figur 1a auch, sowohl auf der ersten Bauteiloberfläche als auch auf der restlichen Oberfläche des Grundkörpers 10 befinden. Es können sich auch Befestigungselemente 20 auf der weiteren Bauteiloberfläche 50 befinden (hier nicht sichtbar). Die Anordnung, Zahl und Form der Befestigungselemente 20 ist, wie bei dem Bauteil 100 aus Figur 1a, frei wählbar.

Figur 2b zeigt ein Bauteil 100 mit einer zylindrischen Form sowie drei elektrischen Leitungselementen 30, die sich von der ersten Bauteiloberfläche 40 zu der weiteren Bauteiloberfläche 50 erstrecken. Es können auch mehr oder weniger als drei elektrische Leitungselemente 30 sein. Wie in Figur 1b ist das eine Befestigungselement 20 als Ring um den Grundkörper 10 ausgestaltet. Auf diese Weise wird eine kreisförmige Kontaktfläche 60 des Befestigungselementes 20 gebildet.

Die Befestigungselemente 20 aus den Figuren 1a bis 2b, übernehmen die Funktion, das Bauteil 100 an einen weiteren Gegenstand, wie beispielsweise ein Gehäuse einer Vorrichtung zu befestigen. Weiterhin sind die Befestigungselemente 20 aus Figur 1b und 2b weiterhin dazu geeignet eine Verbindung zwischen dem Bauteil und dem Gegenstand herzustellen, die hermetisch dicht ist.

Figur 3 zeigt eine erfindungsgemäße Vorrichtung 120 mit einem erfindungsgemäßen Bauteil 100. Das erfindungsgemäße Bauteil 100, das über die Kontaktfläche 60 des Befestigungselements 20 mit dem Gehäuse 130 verbunden ist, weist beispielhaft drei elektrische Leitungselemente 30, mit jeweils einem ersten Ende 32 und einem weiteren Ende 34 auf. Die ersten Enden 32 weisen zum Außenbereich 150 der Vorrichtung 120 hin, während die weiteren Enden 34 zum Innenbereich 140 der Vorrichtung 120 weisen. Das Bauteil 100 ist über die Kontaktfläche 60 des ringförmigen Befestigungselements 20 an die Gehäusewand 160 des Gehäuses 130 der Vorrichtung 120 verbunden. Das Gehäuse 130 weist zur Aufnahme des Bauteils 100 eine Aussparung 190 auf. In die Aussparung wird das Bauteil so eingeführt, dass das Befestigungselement durch beispielsweise Schweißen direkt mit der Gehäusewand 160 so verbunden wird, dass eine hermetisch dichte Verbindung zwischen Gehäuse 130 und Bauteil 100 entsteht. Insbesondere, wenn es sich bei der Vorrichtung um eine medizinische Vorrichtung handelt, wie hier um einen Herzschrittmacher mit einer Batterie 70, die zum Einbringen in einen lebenden Körper geeignet sein soll, ist die erste Oberfläche 170, die die Außenoberfläche der Vorrichtung darstellt biokompatibel sein. Auch der Grundkörper 10 sowie die Teile der elektrischen Leitungselemente 30, die mit dem Körper in Kontakt kommen können, sind aus biokompatiblem Material. Die weitere Gehäuseoberfläche 180 hingegen braucht nicht aus einem biokompatiblen Material gefertigt sein, da sie mit dem Körper nicht in Kontakt tritt.

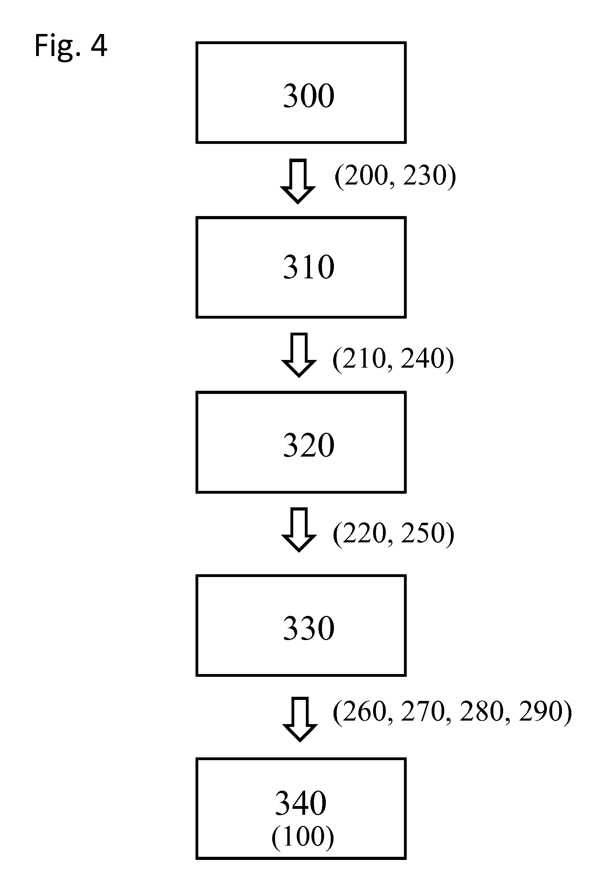

In Figur 4 ist schematisch der Ablauf der Verfahrensschritte des erfindungsgemäßen Verfahrens dargestellt. In einem Schritt I. 300 wird eine erste Masse 200 zur Formung eines ersten Teilbereiches 230, einem Grundgrünkörper, der später den Grundkörper 10 bildet, bereitgestellt. Die erste Masse 200 weist beispielsweise die unter Beispiel 1 angegebene Zusammensetzung auf. In einem Schritt II. 310 wird eine weitere Masse 210 bereitgestellt, um den weiteren Teilbereich 240, einen Cermetgrünkörper, der später das mindestens eine elektrische Leitungselement 30 bildet, zu formen. Die weitere Masse 210 weist beispielsweise die unter Beispiel 2 angegebene Zusammensetzung auf. In einem Schritt III. 320 wird eine dritte Masse 220 zur Formung eines dritten Teilbereiches 250, einem Befestigungsgrünkörper, das später das Befestigungselement 20 bilden wird, bereitgestellt. Die dritte Masse 220 weist beispielsweise die unter Beispiel 3 angegebene Zusammensetzung auf. Die Schritte I. bis III. können nacheinander oder auch gleichzeitig erfolgen. In diesem Beispiel erfolgt das Bereitstellen in den Schritten I. bis III. durch ein Aufschmelzen der Massen 200, 210 und 220. Hierbei wird die erste Masse 200 in eine erste Form aus einer Aluminiumoxidkeramik gegossen. Anschließend wird die weitere Masse 210 in eine weitere Form, wie für die erste Masse 200 beschrieben, gegossen. Die weitere Form weist ein Loch in seinem Inneren auf, das sich mit den Dimensionen des Cermetgrünkörpers deckt. Weiterhin weist die weitere Form eine umlaufende Feder zum Abbilden einer Nut in der äußeren Kontaktfläche des Grundgrünkörpers auf. Ebenso wird die dritte Masse 220 in eine dritte Form, wie für die erste Masse 200 beschrieben, gegossen. Nachdem die Massen 200, 210 und 220 in die jeweiligen Formen gegossen wurden, werden sie für mindestens 4 Stunden bei Raumtemperatur ruhen gelassen, damit sie sich verfestigen. Dies geschieht in Schritt IV. 330 wobei aus dem ersten Teilbereich 230 ein erster Vorläufer 270 für den Grundkörper 10 gebildet wird, sowie aus dem weiteren Teilbereich 240 ein weiterer Vorläufer 280 für das oder die elektrischen Leitungselemente 30, und aus dem dritten Teilbereich 250 ein dritter Vorläufer 290 für das Befestigungselement 20 gebildet. Zusammen wird so aus den Teilbereichen 230, 240 und 250 ein Bauteilvorläufer 260 gebildet.

Nach dem Verfestigen werden die jeweiligen Grünköper aus der Form genommen. Der Cermetgrünköper wird anschließend in das passende Loch im Grundgrünkörper eingebracht, sodass die Enden des Cermetgrünkörpers auf beiden Seiten des Grundgrünkörpers von beiden Seiten des Grundgrünkörpers gleichermaßen gut zugänglich sind. Der Befestigungsgrünkörper wird in die umlaufende Feder des Grundgrünkörpers als Nut und Feder eingebracht.

Der so entstandene Vorläufer für ein Bauteil wird in Schritt V. bei 1600 °C in einem Kammerofen der Firma Nabertherm GmbH über 2,25 Stunden gesintert. Nach 2,25 Stunden wird der Ofen abgeschaltet und das entstandene Bauteil bei geschlossenem Ofen auf Raumtemperatur abkühlen gelassen. Aus dem Cermetgrünkörper ist somit das elektrische Leitungselement 20 mit der Zusammensetzung aus Beispiel 5 entstanden. Aus dem Grundgrünkörper ist der Grundkörper 10 mit der Zusammensetzung aus Beispiel 4 entstanden. Aus dem Befestigungsgrünkörper ist das Befestigungselement 30 mit der Zusammensetzung aus Beispiel 6 entstanden. Das so gebildete Bauteil kann weiterverwertet werden, beispielsweise durch Einbau in das Gehäuse 190 einer Vorrichtung 120.

**Bezugszeichenliste:**

| | | | |
|---|---|---|---|
| 10 | Grundkörper | 220 | dritte Masse |
| 20 | Befestigungselement | 230 | erster Teilbereich |
| 30 | elektrisches Leitungselement | 240 | weiterer Teilbereich |
| 32 | erstes Ende | 250 | dritter Teilbereich |
| 34 | weiteres Ende | 260 | Bauteilvorläufer |
| 40 | erste Bauteiloberfläche | 270 | erster Vorläufer |
| 50 | weitere Bauteiloberfläche | 280 | weiterer Vorläufer |
| 60 | Kontaktfläche | 290 | dritter Vorläufer |
| 70 | Batterie | 300 | Schritt I. |
| 100 | Bauteil | 310 | Schritt II. |
| 110 | Oberfläche des Befestigungselements | 320 | Schritt III. |
| 120 | Vorrichtung, med. Vorrichtung | 330 | Schritt IV. |
| 130 | Gehäuse | 340 | Schritt V. |
| 140 | Innenbereich | | |
| 150 | Außenbereich | | |
| 160 | Gehäusewand | | |
| 170 | erste Gehäuseoberfläche | | |
| 180 | weitere Gehäuseoberfläche | | |
| 190 | Aussparung | | |
| 200 | erste Masse | | |
| 210 | weitere Masse | | |

## Patentansprüche

1. Ein Bauteil (100), beinhaltend:
i. einen Grundkörper (10) mit einer ersten Bauteiloberfläche (40), und einer weiteren Bauteiloberfläche (50), wobei der Grundkörper (10) mindestens zu 50 Gew.-%, bezogen auf das Gesamtgewicht des Grundkörpers (10), eine Keramik beinhaltet;
ii. mindestens ein elektrisches Leitungselement (30), wobei das mindestens eine elektrische Leitungselement (30) mindestens zu 51 Gew.-%, bezogen auf das elektrische Leitungselement (30), ein Metall beinhaltet,
wobei das mindestens eine elektrische Leitungselement (30) durch den gesamten Grundkörper (10) hindurch von der ersten Bauteiloberfläche (40) zu der weiteren Bauteiloberfläche (50) führt;
iii. mindestens ein Befestigungselement (20) mit einer Kontaktfläche (60), wobei das mindestens eine Befestigungselement (20) mindestens zu 51 Gew.-%, bezogen auf das Gesamtgewicht des Befestigungselements (20), ein Metall beinhaltet;
wobei das Befestigungselement (20) mindestens zu einem Teil von dem Grundkörper (10) umgeben ist.

2. Das Bauteil (100) nach Anspruch 1, wobei mindestens 50 % der Oberfläche (110) des mindestens einen Befestigungselementes (20) von dem Grundkörper (10) umgeben ist.

3. Das Bauteil (100) nach Anspruch 1 oder 2, wobei das Befestigungselement (20) ring-förmig ausgestaltet ist.

4. Das Bauteil (100) nach einem der vorhergehenden Ansprüche, wobei das Befestigungselement (20) keine Verbindung durch den Grundkörper (10) hindurch von der ersten Bauteiloberfläche (40) zu der weiteren Bauteiloberfläche (50) bildet.

5. Das Bauteil (100) nach einem der vorhergehenden Ansprüche, wobei der Grundkörper (10) mit dem mindestens einen elektrischen Leitungselement (30) und dem mindestens einen Befestigungselement (20) stoffschlüssig verbunden ist.

6. Das Bauteil (100) nach einem der vorhergehenden Ansprüche, wobei die Keramik des Grundkörpers (10) ausgewählt ist aus der Gruppe bestehend aus Aluminiumoxid (Al₂O₃), Zirkoniumdioxid (ZrO₂) einem ein Aluminiumoxid enthaltendes Zirkoniumoxid (ATZ), einem ein Zirkoniumoxid enthaltendes Aluminiumoxid (ZTA), einem ein Yttrium enthaltendes Zirkoniumoxid (Y-TZP), Aluminiumnitrid (AlN), Titannitrid (TiN), Magnesiumoxid (MgO), einer Piezokeramik, Barium(Zr, Ti)oxid, Barium(Ce, Ti)oxid und Natrium-Kalium-Niobat oder einer Mischung aus mindestens zwei hiervon.

7. Das Bauteil (100) nach einem der vorhergehenden Ansprüche, wobei das Metall des Befestigungselementes (20) und / oder des elektrischen Leitungselementes (30) ausgewählt ist aus der Gruppe bestehend Palladium (Pd), Platin (Pt), Gold (Au), Eisen (Fe), Edelstahl (AISI 304, AISI 316 L), Iridium (Ir), Niob (Nb), Molybdän (Mo), Wolfram (W), Titan (Ti), Kobalt (Co), Chrom (Cr), eine Kobalt-Chrom-Legierung, Tantal (Ta) und Zirkonium (Zr) oder einer Mischung aus mindestens zwei hiervon.

8. Das Bauteil (100) nach einem der vorhergehenden Ansprüche, wobei der Metallgehalt des Befestigungselements (20) größer ist als der Metallgehalt des elektrischen Leitungselementes (30).

9. Das Bauteil (100) nach einem der vorhergehenden Ansprüche, wobei der Metallgehalt des elektrischen Leitungselements (30) größer ist als der Metallgehalt des Befestigungselementes (20) aufweist.

10. Das Bauteil (100) nach einem der vorangehenden Ansprüche, wobei das Bauteil (100) mindestens ein elektrisches Leitungselement (30) aufweist.

11. Das Bauteil (100) nach einem der vorhergehenden Ansprüche, wobei zumindest ein Teil einer der Bauteiloberflächen (40, 50) biokompatibel ist.

12. Eine Vorrichtung (120), beinhaltend:
a. ein Gehäuse (130), das einen Innenbereich (140) von einem Außenbereich (150) mindestens zu einem Teil trennt, beinhaltend:
i). eine Gehäusewand (160) mit einer ersten Gehäuseoberfläche (170), die dem Innenbereich (140) zugewandt ist und einer weiteren Gehäuseoberfläche (180), die dem Außenbereich (150) des Gehäuses (130) zugewandt ist;
ii). mindestens eine Aussparung (190) in der Gehäusewand (160);
b. ein Bauteil (100), gemäß einem der Ansprüche 1 bis 11.

13. Die Vorrichtung (120) nach dem vorhergehenden Anspruch, wobei die Gehäusewand (160) mindestens 30 Gew.-%, bezogen auf das Gesamtgewicht des Gehäuses (130) Titan beinhaltet.

14. Die Vorrichtung (120) nach einem der beiden vorhergehenden Ansprüche, wobei zumindest ein Teil des Bauteils (100), der dem Außenbereich (150) zugewandt ist und zumindest der Teil der Gehäuseoberfläche (170, 180), die dem Außenbereich (150) zugewandt ist, biokompatibel ist.

15. Ein Verfahren zur Herstellung eines Bauteils (100) beinhaltend mindestens folgende Schritte:
I. Bereitstellen einer ersten Masse (200) zur Formung eines ersten Teilbereiches (230);
II. Bereitstellen einer weiteren Masse (210) zur Formung mindestens eines weiteren Teilbereiches (240);
III. Bereitstellen einer dritten Masse (220) zur Formung eines dritten Teilbereiches (250);
IV. Bilden eines Bauteilvorläufers (260), wobei ein erster Vorläufer (270) für einen Grundkörper (10) aus dem ersten Teilbereich (230) gebildet wird, wobei ein weiterer Vorläufer (280) für ein elektrisches Leitungselement (30) aus dem mindestens einen weiteren Teilbereich (240) gebildet wird und wobei ein dritter Vorläufer (290) für mindestens ein Befestigungselement (20) aus dem dritten Teilbereich (250) gebildet wird;
V. Behandeln des Bauteilvorläufers (260) bei einer Temperatur in einem Bereich von 500 bis 2300 °C;
wobei sich der weitere Vorläufer (280) durch den gesamten ersten Vorläufers (270) hindurch erstreckt,
wobei mindestens ein Teil der dritten Masse (220) von einem Teil der ersten Masse (200) umgeben ist.

16. Das Verfahren nach Anspruch 15, wobei Schritt IV. einen Formgebungsprozess beinhaltet, bevorzugt ausgewählt aus der Gruppe bestehend aus einem lithographischen Prozess, einem Spritzgießen, einem Zerspanen, einem Extrudieren oder einer Kombination von mindestens zwei hiervon.

17. Das Verfahren nach einem der Ansprüche 15 oder 16, wobei in Schritt IV. ein Behandeln des Bauteilvorläufers (260) bei einer Temperatur in einem Bereich von 120 bis 500 °C vor dem Behandeln in Schritt V. erfolgt.

18. Das Verfahren nach einem der Ansprüche 15 bis 17, wobei die erste Masse zur Formung des ersten Teilbereiches und Bildung des ersten Vorläufers (270) für den Grundkörper (10) so ausgestaltet ist, dass sie im Behandlungsschritt V. um mindestens 1 %, bezogen auf das Volumen des ersten Vorläufers (270) mehr schrumpft als die weitere Masse (210) zur Formung des weiteren Vorläufers (240) für das Leitungselement (30), bezogen auf das Volumen des weiteren Vorläufers (240).

19. Ein Bauteil (100) erhältlich nach einem Verfahren gemäß mindestens einem der Ansprüche 15 bis 18.

20. Eine Vorrichtung (120) beinhaltend mindestens ein Bauteil (100) nach einem der Ansprüche 1 bis 11 oder 19.

21. Die Vorrichtung (120) nach Anspruch 20, wobei die Vorrichtung (120) mindestens ein Gehäuse (130) mit mindestens einer Aussparung (190) und mindestens einem Bauteil (100) nach einem der Ansprüche 1 bis 11, oder 19 beinhaltet, wobei das Bauteil (100) über das mindestens eine Befestigungselement (20) mit dem Gehäuse (90) verbunden ist, wobei durch das Bauteil (100) mindestens eine elektrische Verbindung zwischen mindestens einem Innenbereich (140) des Gehäuses (90) und mindestens einem Außenbereich (150) hergestellt ist.
